# EUROPEAN PATENT APPLICATION

(11) **EP 3 603 587 A1**
(43) Date of publication of application: **05.02.2020**
(21) Application number: 18776634.0
(22) Date of filing: 17.01.2018
(51) Int. Cl.: A61F 5/14

(54) **METHOD FOR MANUFACTURING AN ORTHOPAEDIC INSOLE AND PRODUCT THUS OBTAINED**

(30) Priority: 31.03.2017 ES 201730204
(71) Applicant: Voxelcare, SL, 03202 Elche (ES)
(72) Inventor: LEDESMA LATORRE, Santiago, 03600 Elda (ES)
(74) Representative: Juncosa Miró, Jaime
(86) International application number: PCT/ES2018/070034
(87) International publication number: WO 2018/178426

(57) **Abstract**

The invention relates a method for manufacturing an orthopaedic insole and to a product thus obtained, the method comprising:
- a first step (1) of 3D modelling a future insole with a specific geometry and which will ideally be formed by a soft surface (10) and a rigid surface (11);
- a second step (2) of software treatment for the "flattening" or modelling of the original soft upper part (10), transforming same into a soft surface or element (10a) obtained with its lower surface being completely flat and the relative thicknesses still being maintained;
- a third step (3) of manufacturing the soft surface (10a) obtained in the second step (2) by means of milling or additive manufacturing techniques;
- a fourth step (4) of joining the obtained soft surface (10a) to the rigid surface (11), obtaining a sandwich-type insole.

## Description

### Object of the Invention

The object of the present specification is a method for manufacturing an orthopaedic insole and the product thus obtained, whereby the upper part of said insole is geometrically transformed for the purpose of allowing its production by means of milling or additive manufacturing techniques.

### Background of the Invention

At present, an orthosis, according to the definition of the International Organisation for Standardisation (ISO), is a support or another external device (apparatus) applied to the body for modifying functional or structural aspects of the neuromuscular and skeletal system.

The term is used to refer to apparatuses or devices, splints, technical aids and supports used in orthopaedics, physiotherapy, and occupational therapy which correct or facilitate the performance of an action, activity, or movement, seeking to save energy and increase safety. They are used to hold, align, or correct deformities and to improve the function of the locomotor system.

Orthosis are different from prostheses as they do not partially or completely substitute an organ or limb with physical incapacity, disability, or dysmetry, but rather take over or reinforce its functions.

More specifically, insoles, which are elements placed inside shoes and used as an interface between feet and shoes, fall within said field, and the purpose of the insoles relates to the use thereof as a therapeutic measure in a plethora of pathologies ranging from the treatment of diabetes-associated neuropathies to the correction of dysmetries or the walking pattern.

There are many types of different orthopaedic insoles and manufacturing techniques. Different devices are used depending on the type of pathology, although they can basically be classified into rigid insoles with a soft upper lining or insoles entirely manufactured with soft or flexible materials.

The insoles perform their therapeutic action using two fundamental principles: redistribution of pressure and change in foot orientation in the different phases of walking.

The redistribution of pressure is carried out by means of adding elements to the upper part of the insole, such as metatarsal bars, subcalcaneal unweighting elements, fascial accommodations, etc. The idea is that when there is a point on which a large amount of pressure is exerted while walking, that excessive pressure can be reduced by elevating other areas of the insole so that the area of contact of the foot increases or reducing the thickness in the area on which excessive pressure is exerted.

The change in the walking pattern is carried out by means of modifications in the orientation of the insole, changes in the height and geometry of the arch, or inclined planes in different areas which force the foot to have a different orientation.

The redistribution of pressure is performed by means of soft insoles or linings made of materials such as EVA to which modules or elements with specific shapes are incorporated. The change in the walking pattern can also be performed in insoles manufactured entirely with flexible materials, although the use of rigid bases generally offers a better result.

In the more general case, an insole can be seen as a "sandwich" or multilayer structure having a rigid base that provides support and allows changing the orientation of the foot during the different phases of walking, and a soft surface on which treatments for the redistribution of pressure are performed.

The manufacturing of said insoles was conventionally performed by means of filling a mould of the patient's foot with plaster, subsequently modifying this mould, and performing series of material thermoforming and sanding steps.

Today, a high percentage of insoles or orthoses are manufactured by means of CAD/CAM techniques, in which a design software, based on the geometry of the foot and other complementary information, allows obtaining a 3D geometry that will then be manufactured or manufactured by means of additive techniques, also known as 3D printing.

This type of manufacturing technology is applicable both to rigid insoles, generally milled in materials such as polypropylene, and soft insoles, generally milled from blocks of materials such as EVA rubber.

However, the manufacturing of "sandwich"-type insoles, where treatments for redistribution of pressure are combined with treatments for change in the walking pattern, cannot be carried out today by means of CAD/CAM techniques.

In the case of manufacturing the lower (rigid) part by means of CAD/CAM, the pressure redistribution modules located in the upper part must be placed by hand in the orthosis production workshop. The reason for this is that it is not possible to manufacture a soft insole or orthosis with a 3D geometry on both faces by means of milling.

Some examples of what has been expressed at this point include patent ES 2 336 533 which describes a process for capturing the plantar image through a device with elastic membrane securing and tensioning means, as well as double-sided machining of the insole obtained in said process, the process being characterized by comprising the following steps:
(a) positioning the securing and tensioning means, made up of two adjustable horizontal bars, on the scanner,
(b) adjusting the height and distance between said horizontal bars,
(c) placing the membrane on the securing means;
(d) securing the membrane through T-shaped platens adhered to the horizontal bars;
(e) adjusting the tension of the membrane through a crank placed on the side of one of the horizontal bars;
(f) placing the foot on the membrane and capturing the image;
(g) performing double-sided machining of the insole through the image of the surface given in STL format.

Another example is patent ES 2 021 968 which describes an orthopaedic insole device for providing proper support for a patient's foot within a shoe, provided with a top surface for contacting the foot and a bottom surface for contacting a shoe insole, comprising: a shaped monolithic orthotic material formed in its top surface with a plurality of closely spaced, substantially parallel grooves whereby slippage of the foot relative to the orthotic device is controlled.

Likewise, patent WO/2003/015670 describes an improved method of producing insoles that are used to correct foot deformities, comprising a sequence of process steps which begin with a prior diagnosis by a specialist, consisting of the examination of the feet in order to assess and, if the patient so requires, to correct the somatic deficiency thereof by means of surgery or by means of an orthotic insole after having obtained the diagnosis, in order to proceed to the production of the orthotic insole, characterised in that the method comprises:
- a first step consisting of elaborating a paper pattern of the footprint of the patient where the print of the correction which will be made has been left, for which lines denoting the limit up to where the insole will have to reach are drawn on the foot of the patient using a non-erasable ink pen, separately proceeding to trace a pattern of the inside the shoe normally used by the patient, which is cut out and placed inside the shoe, subsequently dipping the paper pattern in alcohol and which will print the drawing made on the sole of the foot onto the paper in a similar way to a stencil, for which the patient is asked to put his or her shoes back on, being careful not to let the insole smudge;
- a second step consisting of the preparation of the orthotic insole consisting of again obtaining the pattern of the insole on a second piece of paper, copying the part which must be corrected, drawing the contour of said cut-out shape onto a resin sheet of thermo-mouldable plastic, and wherein one side of which is smooth whereas the other side is corrugated by a loosely woven fabric layer, this material serving as reinforcement, producing the base of the insole by cutting out the contour of the shape drawn on the surface of the thermo-mouldable resin sheet and placing it in a kiln according to the specific temperature of each material for the purpose of softening it, the temperature of which kiln ranges between 35 and 120°C during a period of 2 to 3 minutes;

- a third step consisting of preparing the feet of the patient to receive the chosen thermo-mouldable resin sheet, placing a disposable plastic sock, with which the sock of the patient is separated both as a measure of hygiene between the doctor and the patient and in order to perfectly isolate the foot, said element being very simple and economical, in order to subsequently place a heat-insulating sock, the function of which will be to prevent any harm to the sole of the foot of the patient due to heat, and then a hose connected to a vacuum pump in the sock, the function of which will be to extract all of the air;
- a fourth step consisting of placing a thermo-mouldable resin pre-softened in the kiln on the heat-insulating sock so that the resin does not stick to the sock, inserting a paper film between the sock and the softened resin, covering all the elements with a plastic bag and sealing the bag with neoprene straps with a fastener such as clasps or an adhesive Velcro film until observing the cooling of the resin moulded directly on the foot of the patient;
- a fifth step consisting of the smoothening and lining of the moulded orthotic insole, consisting of sending the latter to the orthopaedic workshop for the smoothening and lining thereof, where the corresponding unweighting elements are incorporated depending on the specific case, using usual tools and machinery for the production of orthotics in the final finishing.

None of the documents described above solves the problems of obtaining an orthopaedic insole or orthosis formed by a rigid base and a soft surface with different reliefs to perform redistributions of pressure.

This is due to the fact, with the current knowledge of the state of the art, those insoles which may be formed by a rigid base and a soft upper part (such as the one proposed in the present invention) would have to incorporate in their soft base a 3D shape both in their upper part and in their lower part, so it must be manufactured by means of double-sided milling to achieve said objective.

However, this solution means that, while milling one side, given that it is a soft, non-rigid material, the other side may not be machined because the material, instead of being milled, is moved by the tool.

Therefore, a (previously attempted) way for machining this soft part consists of sticking a block of soft material on an orthosis or rigid part for subsequent milling in a machine tool.

The lower surface of the soft part therefore takes the shape of the upper surface of the rigid part, and only the upper surface of the soft part has to be milled. However, in this case, there is a need to know with great precision the position of the orthosis in the space and to have specific fastening systems that vary with each foot shape and size, so the manufacture must be done practically according to the needs of each user, which would turn the obtained insole into a rather unaffordable product.

### Disclosure of the Invention

The technical problem solved by the present invention is to achieve an orthopaedic insole formed by a rigid base and a soft surface, incorporating a series of reliefs to perform redistributions of pressure while walking and allowing the production thereof by means of milling or additive manufacturing techniques, which allow mass manufacturing, reducing the cost for manufacturing and subsequently selling the obtained product (insole).

As a result of the method for manufacturing herein described, orthopaedic insoles or orthoses can be made by means of machinery present in the state of the art, eliminating the need to have a workshop with sanding and gluing systems and a stock of parts that do not allow making unweighting elements or surface changes for the redistribution of pressure.

With this production model, sandwich-type orthopaedic insoles or orthoses can be manufactured within a shorter time with greater precision in element positioning as it allows using 3D modelling techniques that are only available when the insole is not made by means of sandwich-type method.

Throughout the description and claims, the word "comprises" and variants thereof do not seek to exclude other technical features, additions, components, or steps. For those skilled in the art, other objects, advantages, and features of the invention will be inferred in part from the description and in part from putting the invention into practice. The following examples and drawings are provided by way of illustration and do not seek to limit the present invention. Furthermore, the present invention covers all the possible combinations of particular and preferred embodiments herein indicated.

### Brief Description of the Drawings

A series of drawings that help to better understand the invention and are expressly related to an embodiment of said invention, presented as a non-limiting example thereof, is described very briefly below.
Figure 1 shows a schematic view of the method for manufacturing an orthopaedic insole.
Figure 2 shows a view of the elements forming the insole obtained by means of the method herein described.
Figure 3 shows a view of the orthopaedic insole obtained by means of the method for manufacturing herein described.

### Preferred Embodiment of the Invention

The attached drawings show a preferred embodiment of the invention. More specifically, the method for manufacturing an orthopaedic insole, object of the present specification, comprises a first step 1 of 3D modelling a future insole with a specific geometry and which will ideally be formed by a soft surface 10 and a rigid surface 11.

A second step 2 of software treatment for the "flattening" or modelling of the original soft upper part 10, transforming same into an obtained soft surface or element 10a with its lower surface being completely flat and the relative thicknesses still being maintained, is then performed.

To that end, the software uses mathematical models based on finite element and graph theories, where the structure of the flat upper flat is modelled, modelling a soft object the lower part of which is flat.

This is achieved by means of performing calculation using iterative methods based on the resolution of non-linear systems with multiple unknowns, the position of each of the points of the lower surface, such that all of them share the same height (Z coordinate) and the distance between them, which they had in their original three-dimensional position, is respected. A large amount of equations is introduced in said calculation method, where the position of each of the points represents an unknown, and the restrictions associated with the known distances between the points represent equations to be solved. To that end, techniques for the resolution of non-linear systems of equations are implemented by means of conjugate-gradient resolution techniques, such as Stanford University's LSQR method, for example (http://web.stanford.edu/group/SOUsoftware/lqsr/).

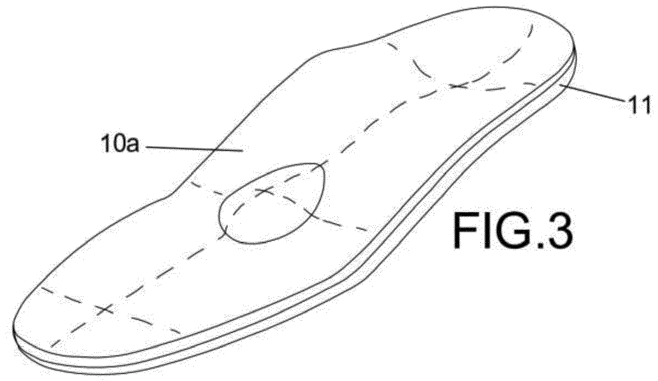

The method comprises a third step 3 for manufacturing the obtained soft surface 10a obtained in the second step 2 by means of milling or additive manufacturing techniques and a fourth step 4 of joining the obtained soft surface 10a to the rigid surface 11, obtaining a sandwich-type insole, as shown in Figure 3.

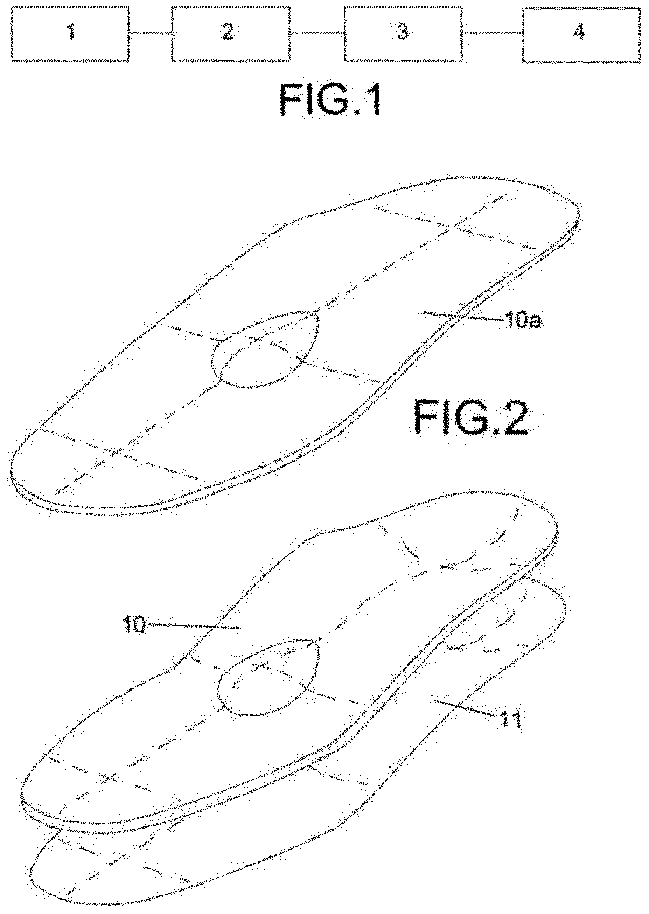

In a preferred embodiment, the insole is formed by the soft surface or element 10 and the rigid surface or element 11 which are ideally designed as shown in Figure 2.

The way to machine the original soft surface 10 is complicated as it has a curvature on top and another one at the bottom. There is obtained in the second step an obtained soft surface 10a which in turn contains a flat lower surface and is capable of obtaining a geometry identical to the geometry of the original soft surface 10 once said obtained soft surface 10a is joined to the rigid surface 11, bending and curving as it is formed from a soft material, as well as obtaining a geometry identical to the geometry of the original soft surface 10. This can be seen in the images shown in Figure 2, where the dashed lines shown have the same length both in the original soft surface 10 and in the obtained soft surface 10a.

## Claims

1. A method for manufacturing an orthopaedic insole, the method comprising:
- a first step (1) of 3D modelling a future insole with a specific geometry and which will ideally be formed by a soft surface (10) and a rigid surface (11), the method being **characterized by** further comprising:
- a second step (2) of software treatment for the "flattening" or modelling of the original soft upper part (10), transforming same into an obtained soft surface or element (10a) with its lower surface being completely flat and the relative thicknesses still being maintained;
- a third step (3) of manufacturing the obtained soft surface (10a) obtained in the second step (2) by means of milling or additive manufacturing techniques; and
- a fourth step (4) of joining the obtained soft surface (10a) to the rigid surface (11), obtaining a sandwich-type insole.

2. An orthopaedic insole obtained according to the method of claim 1, comprising an obtained soft surface (10a) and a rigid surface (11) by way of a sandwich-type insole.
